# EUROPEAN PATENT APPLICATION

(11) **EP 1 506 740 A1**
(43) Date of publication of application: **16.02.2005**
(21) Application number: 03017631.7
(22) Date of filing: 12.08.2003
(51) Int. Cl.: A61B 5/15

(54) **Safety syringe with retractable needle**

(71) Applicant: Chen, Long-Hsiung, Taipei (TW)
(72) Inventor: Chen, Long-Hsiung, Taipei (TW)
(74) Representative: Casalonga, Axel

(57) **Abstract**

A safety syringe with retractable needle is disclosed. The syringe body contains a hollow needle mounted on a base. An ear on the base extends through a guide slot in the wall of the body. The ear follows the guide slot and allows the needle to extend and protrude from the end of the body or be retracted back into the body after use. A front and rear lock slot hold the needle in an extended or retracted position. Once retracted, the needle tilts to one side, thus easily identifying the syringe as used and preventing the needle from accidentally protruding from the syringe body. A vial removal knob provides easy removal of the sample vial. A rear cover closes the end of the body to prevent the rear needle tip from protruding from the body. Therefore, a high degree of safety for medical personnel and patients is ensured.

## Description

### BACKGROUND OF THE INVENTION

### Field of the Invention

The present invention relates to a syringe. More specifically, the present invention discloses a safety syringe with a lockable, retractable, and removable needle for extracting blood samples.

### Description of the Prior Art

In recent years, due to improved living standards, an increasing number of people are paying closer attention to their health. As a result, not only are health care standards being raised, but also the number of examinations is increasing. Medical personnel are now required to perform more examinations and at a higher quality.

During medical treatment, therapy, or rehabilitation it is common for blood samples to be required. In this way, the patient's condition can be more accurately determined. In order to obtain the blood sample medical personnel use various means or methods to extract the blood.

Regardless of the traditional method used, the risk of exposure to germs, viruses, bacteria, or diseases found in blood for patients and medical personnel is high. In the current era of diseases and epidemics such as AIDS, exposure to tainted blood may be fatal.

Using traditional methods, medical personnel may be accidentally poked by exposed needles from used syringes. Additionally, used syringes may inadvertently be used again since they are not easily identifiable as having been used. In this way, a patient may be exposed to another patient's blood. In either of these situations, the outcome could be fatal.

Additionally, the blood sample itself may be contaminated thereby destroying the accuracy of the blood sample analysis. If this happens, the diagnosis may be inaccurate and the patient incorrectly treated. These results could also be fatal for the patient.

Furthermore, conventional methods of extracting blood samples are not as convenient for medical personnel and patients as could be. Therefore, during use the patient may experience discomfort and the medical personnel may experience inconvenience.

Therefore, there is a need for a safe, efficient, and effective safety syringe for extracting blood samples that ensures the safety and increases the convenience for medical personnel and patients.

### SUMMARY OF THE INVENTION

To achieve these and other advantages and in order to overcome the disadvantages of the conventional method in accordance with the purpose of the invention as embodied and broadly described herein, the present invention provides a safety syringe with a retractable and removable needle for safely and efficiently extracting blood samples.

As shown above, health care professionals and patients alike may be exposed to germs or diseases or contaminated with tainted blood when extracting blood samples. If such exposure occurs, death to the patient or health care professional may result.

Additionally, traditional syringes do not provide a high enough level of convenience during use.

Therefore, the present invention provides a safety syringe with retractable needle and locking extended and retracted positions that ensures the safety of medical personnel and patients as well as an improved ease of use.

The safety syringe of the present invention comprises a body with a tip with an opening located on the top of the body. A guide slot is positioned in the side of the body. The guide slot further comprises a front locking slot, a rear locking slot, and a saw tooth section. A cover allows the rear end of the body to be sealed off for safety reasons such as to prevent accidental contact with a rear needle or to prevent contamination from entering the inside of the body. A tab and slot is provided to allow the internal components of the safety syringe to be easily assembled inside the body.

A base with a needle mount, a base slot, and an ear are provided for mounting and holding a needle and allowing efficient movement of the needle assembly. The base slot is provided to allow for appropriate flexibility to the wall of the base. The needle assembly comprises a front needle, a front needle base, a rear needle, and a rear needle base. The front needle base and the rear needle base are designed to mate to allow the two bases to attach together. Therefore, once the two bases are attached together, the front needle and the rear needle act as a single needle so that a continuous hollow tube runs the length of the needle assembly, from the tip of the front needle to the end of the rear needle. The needle assembly mounts to the needle mount on the base. The needle assembly and base are inserted into the body. The ear of the base extends through the guide slot so that the movement of the base and the needle assembly may be easily controlled. The tab and slot allow the wall of the body to be flexible during assembly. Once the internal components are assembled into the body, the tab and slot are pressed together to increase the rigidity of the wall of the body.

The guide slot in conjunction with the ear guides the movement of the base. When the base is moved to the front of the body to the end of the guide slot, the ear reaches a front locking slot. The front locking slot is provided so that once the ear enters the front locking slot, the needle assembly and base are not allowed to inadvertently retract back into the body. In this way, the needle assembly is maintained in a fixed extended position for use. The saw tooth section improves the safety of the syringe by providing an obstacle for the ear so that the needle assembly does not accidentally move forward through the guide slot and protrude from the body when the ear is not locked in the rear lock slot. The forward locking slot is an L-shaped slot allowing the ear to move to the left or to the right. Using the forward locking slot allows the tip of the front needle to be rotated to a certain angle to the left or right. In certain situations it is useful for the tip of the front needle to be rotated for improved ease of use. Therefore, in various embodiments of the present invention, the front locking slot is formed in different shapes as required.

In preparation for use such as taking a blood sample, the needle assembly and base are moved to the front and the ear is locked in place in the front locking slot. A sample vial is inserted in the body. The sample vial further comprises a seal for preventing contamination to the inside of the sample vial or spillage of contents of the sample vial as well as maintaining a vacuum inside the sample vial. The sample vial is inserted until the tip of the rear needle punctures the seal. The flexible nature of the seal membrane allows for efficiently inserting the needle and at the same time adaptably forming around the rear needle. Once the seal is punctured by the rear needle, the vacuum inside the sample vial along with the patient's blood pressure draws the blood from the patient through the needle assembly and into the sample vial. After the blood sample has been successfully taken, the sample vial is removed from the body of the safety syringe. For ease in removing the sample vial, the ear may be rotated out of the forward locking slot and pulled through the guide slot. After the sample vial has been removed from the safety syringe, the cover is closed.

A vial removal knob is provided on the side of the syringe body. When the sample vial needs to be removed from the safety syringe, a health care profession simply needs to press or push the vial removal knob. An arm of the vial removal knob presses against the top of the sample vial thereby dislodging the sample vial from the base and needle assembly. Once dislodged, the sample vial can be easily grasped and removed.

When the ear is moved to the rear of the guide slot it encounters a rear locking slot. The rear locking slot is angled so that when the ear is in a fully retracted position, the base will tilt to a certain angle. In this way the entire needle assembly, since it is completely inside the body, will slant to one side. This easily identifies the safety syringe as having been used and prevents accidental reuse of the safety syringe and prevents the front needle from protruding from the body of the safety syringe. Since the base comprises a base slot, the wall of the base is flexible and therefore will bend allowing the base to tilt at an angle. As a result, the safety of medical personnel is ensured against accidentally being poked by the tip of the front needle. Also, since the cover closes the rear end of the body, medical personal are not in danger of being poked by the tip of the rear needle. Additionally, used syringes are easily identified and disposed of thereby preventing accidental reuse and ensuring the safety of patients.

The rear locking slot may be angled to the left or right allowing the needle to tilt to the left or right. Alternatively, the rear locking slot is not angled. Therefore, the base and needle assembly will not tilt to an angle when fully retracted. However, the base and needle assembly will still be locked in a retracted position to prevent the front needle from protruding from the body. The guide slot is an arc-shaped path ending in a forward lock slot. In this way, the ear can be easily moved from a retracted position to a fully locked extended position is a single smooth movement. Additionally, the forward lock slot may be positioned to the right instead of to the left.

In the previous description, the nature of the guide slot requires the ear to be moved along the guide slot until it reaches the end and then turned to one side in the forward lock slot. The arc-shaped guide slot allows the ear to smoothly travel the length of the guide slot to the forward lock slot in one motion, thereby increasing the functionality and ease of use of the safety syringe for medical personnel.

In some implementations, the base is an extended base. The function and use are similar to the previously described embodiments. However, in this embodiment the cover is attached to the base instead of to the body. The extended base allows for varying sizes and lengths of sample vials to be used as well as providing coverage around the sample vial when the base is retracted to the rear of the guide slot. After the blood sample has been taken, the needle assembly can be retracted to the fully retracted position in the rear lock slot. The length of the extended base may be made to an appropriate length that is suitable for the sample vial size to be used.

Alternatively, the slot and tab are not provided. Therefore, the body is a solid wall at the rear of the body instead of being split. In order to facilitate assembly of the safety syringe the ear is attachable to the base instead of being manufactured as a part of the base. Once the base is inserted into the body, the ear is attached to the base from the outside of the body. The ear and base comprise appropriate mating tabs or slots to allow the ear to be firmly attached to the base. For example, the base may have a slot and the ear a tab or the base may have a tab and the ear a slot. Once assembled, the function and use of the safety syringe is as previously described.

Thus, the present invention provides a safety syringe with retractable needle and locking extended and retracted positions that ensures the safety of medical personnel and patients as well as an improved ease of use.

These and other objectives of the present invention will become obvious to those of ordinary skill in the art after reading the following detailed description of preferred embodiments.

It is to be understood that both the foregoing general description and the following detailed description are exemplary, and are intended to provide further explanation of the invention as claimed.

### BRIEF DESCRIPTION OF THE DRAWINGS

The accompanying drawings are included to provide a further understanding of the invention, and are incorporated in and constitute a part of this specification. The drawings illustrate embodiments of the invention and, together with the description, serve to explain the principles of the invention. In the drawings,
Figure 1 is an assembly drawing showing a safety syringe according to an embodiment of the present invention;
Figure 2 is a plan view drawing illustrating a safety syringe in an extended position according to an embodiment of the present invention;
Figure 3 is an assembly drawing showing a safety syringe according to an embodiment of the present invention;
Figure 4A is a drawing illustrating a safety syringe according to an embodiment of the present invention;
Figure 4B is a drawing illustrating a safety syringe according to an embodiment of the present invention;
Figure 5A is a plan view drawing illustrating a safety syringe and sample vial according to an embodiment of the present invention;
Figure 5B is a plan view drawing illustrating a safety syringe and sample vial according to an embodiment of the present invention;
Figure 6A is a drawing showing a safety syringe with the needle in an extended position according to an embodiment of the present invention;
Figure 6B is a drawing showing a safety syringe with the needle in a retracted position according to an embodiment of the present invention;
Figure 6C is a drawing showing a safety syringe with the needle in a retracted and slanted position according to an embodiment of the present invention;
Figure 7 is an assembly drawing showing a safety syringe with alternative guide slot according to an embodiment of the present invention;
Figure 8 is a drawing illustrating a safety syringe with extended base according to an embodiment of the present invention;
Figure 9 is a plan view drawing showing a safety syringe with extended base according to an embodiment of the present invention;
Figure 10A is a drawing illustrating a safety syringe with vial removal knob according to an embodiment of the present invention;
Figure 10B is an enlarged view of the vial removal knob according to an embodiment of the present invention;
Figure 11 A is a plan view drawing illustrating a safety syringe with vial removal knob according to an embodiment of the present invention;
Figure 11B is a plan view drawing illustrating a safety syringe with vial removal knob according to an embodiment of the present invention;
Figure 12A is a drawing illustrating a safety syringe with vial removal knob according to an embodiment of the present invention; and
Figure 12B is an enlarged view of the vial removal knob according to an embodiment of the present invention.

### DESCRIPTION OF THE PREFERRED EMBODIMENTS

Reference will now be made in detail to the preferred embodiments of the present invention, examples of which are illustrated in the accompanying drawings. Wherever possible, the same reference numbers are used in the drawings and the description to refer to the same or like parts.

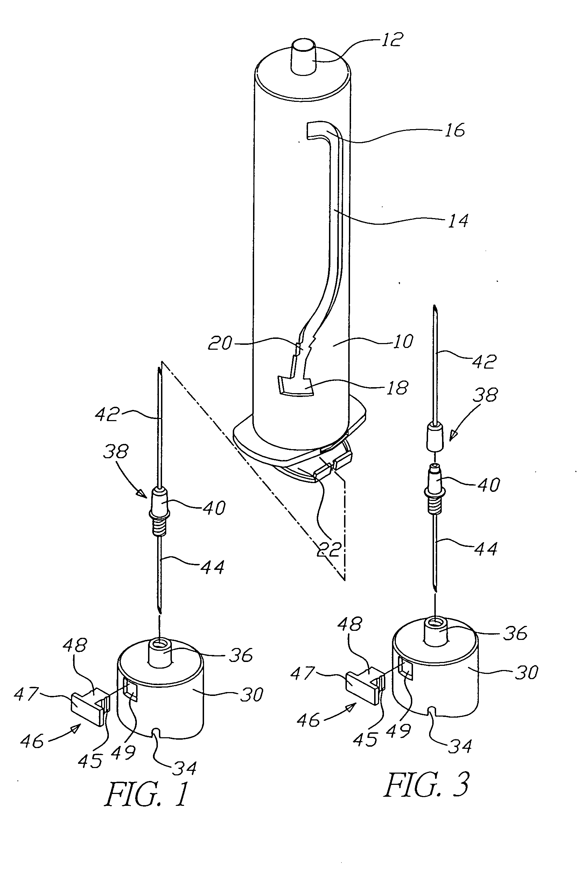

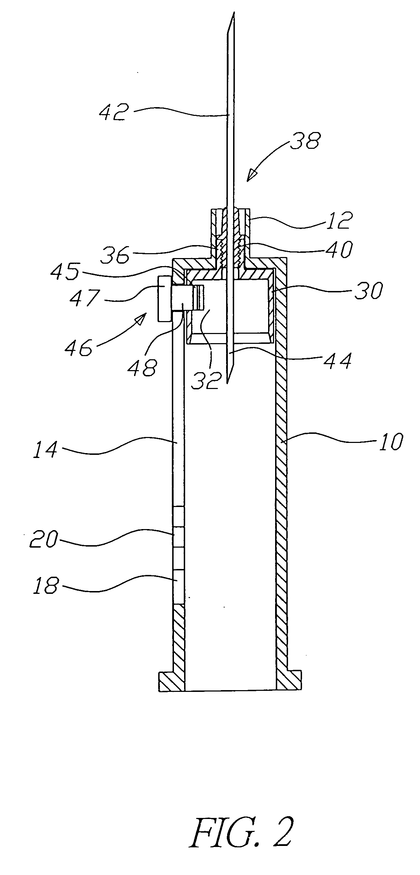

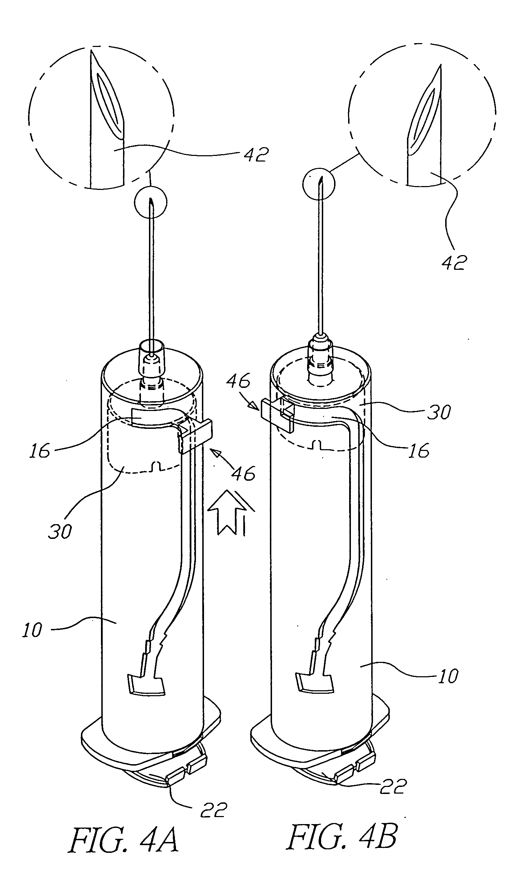

Refer to Figure 1, which is an assembly drawing showing a safety syringe according to an embodiment of the present invention, Figure 2, which is a plan view drawing illustrating a safety syringe in an extended position according to an embodiment of the present invention, and Figure 3, which is an assembly drawing showing a safety syringe according to an embodiment of the present invention.

The safety syringe of the present invention comprises a body 10 with a tip 12 with an opening located on the center of the top of the body 10. A guide slot 14 is positioned in the side of the body 10. The guide slot 14 further comprises a front locking slot 16, a rear locking slot 18, and a saw tooth section 20. A cover 22 allows the rear end of the body 10 to be sealed off for safety reasons such as to prevent accidental contact with a rear needle or to prevent contamination from entering the inside of the body 10.

A base 30 with a needle mount 36, a base slot 34, and an ear 46 are provided for mounting and holding a needle and allowing efficient movement of the needle assembly. The base slot 34 is provided to allow for appropriate flexibility to the wall of the base 30. The ear 46 is removably attached to the base 30. The ear 46 further comprises an ear face 47 and an ear retaining tab 48. The ear retaining tab 48 provides grooves to ensure the ear 46 is held firmly to the base 30 once the ear 46 is inserted into a mating hole 49 in the base 30.

The needle assembly comprises a front needle 42, a front needle base 38, a rear needle 44, and a rear needle base 40. The front needle base 38 and the rear needle base 40 are designed to mate to allow the two bases to attach together. Therefore, once the two bases are attached together, the front needle 42 and the rear needle 44 act as a single needle so that a continuous hollow tube runs the length of the needle assembly, from the tip of the front needle 42 to the end of the rear needle 44.

The needle assembly mounts to the needle mount 36 on the base 30. To facilitate mounting, the rear needle base 40 is threaded to match threads in the needle mount 36. The needle assembly and base 30 are inserted into the body 10. Once the base 30 is inside the body 10, the ear 46 is snapped into the mating hole 49 of the base 35. The ear 46 of the base 30 extends through the guide slot 14 so that the movement of the base 30 and the needle assembly may be easily controlled.

The guide slot 14 in conjunction with the ear 46 guides the movement of the base 30. When the base 30 is moved to the front of the body 10 to the end of the guide slot 14, the ear 46 reaches a front locking slot 16. The front locking slot 16 is provided so that once the ear 46 enters the front locking slot 16, the needle assembly and base 30 are not allowed to inadvertently retract back into the body 10. In this way, the needle assembly is maintained in a fixed extended position for use.

The saw tooth section 20 improves the safety of the syringe by providing an obstacle for the ear so that the needle assembly does not accidentally move forward through the guide slot 14 and protrude from the body 10 when the ear is not locked in the rear lock slot.

Refer to Figure 4A, which is a drawing illustrating a safety syringe according to an embodiment of the present invention, and Figure 4B, which is a drawing illustrating a safety syringe according to an embodiment of the present invention.

In an embodiment of the present invention the forward locking slot 16 is an L-shaped slot allowing the ear 44 to move to the left. In other embodiments of the present invention the forward locking slot 16 is positioned to the right. The forward locking slot 16 allows the tip of the front needle 42 to be rotated to a certain angle to the left or right. In certain situations it is useful for the tip of the front needle 42 to be rotated for improved ease of use. Therefore, in various embodiments of the present invention, the front locking slot is formed in different shapes as required.

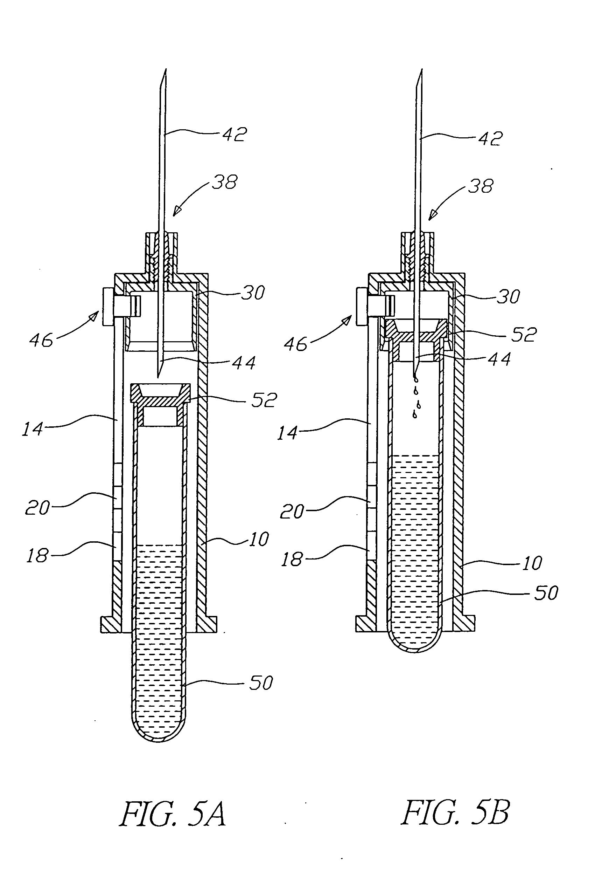

Refer to Figure 5A, which is a plan view drawing illustrating a safety syringe and sample vial according to an embodiment of the present invention, and Figure 5B, which is a plan view drawing illustrating a safety syringe and sample vial according to an embodiment of the present invention.

In preparation for use such as taking a blood sample, the needle assembly and base 30 are moved to the front and the ear 46 is locked in place in the front locking slot 16. A sample vial 50 is inserted in the body 10. The sample vial 50 further comprises a seal 52 for preventing contamination to the inside of the sample vial 50 or spillage of contents of the sample vial 50 as well as maintaining a vacuum inside the sample vial 50. The sample vial 50 is inserted until the tip of the rear needle 44 punctures the seal 52. The flexible nature of the seal 52 membrane allows for efficiently inserting the needle and at the same time adaptably forming around the rear needle 44. Once the seal 52 is punctured by the rear needle 44, the vacuum inside the sample vial 50 along with the patient's blood pressure draws the blood from the patient through the needle assembly and into the sample vial 50.

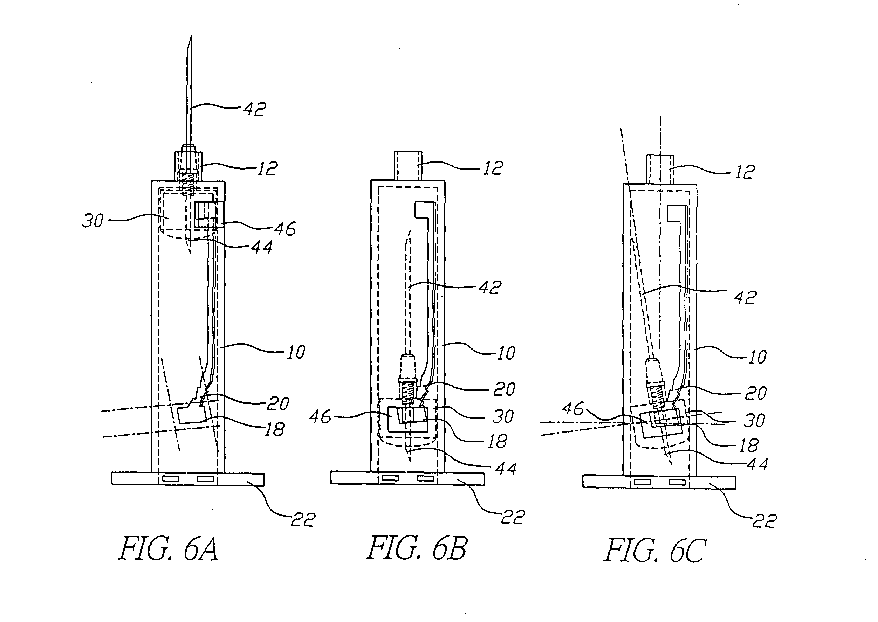

Refer to Figure 6A, which is a drawing showing a safety syringe with the needle in an extended position according to an embodiment of the present invention, Figure 6B, which is a drawing showing a safety syringe with the needle in a retracted position according to an embodiment of the present invention, and Figure 6C, which is a drawing showing a safety syringe with the needle in a retracted and slanted position according to an embodiment of the present invention.

After the blood sample has been successfully taken, the sample vial is removed from the body 10 of the safety syringe. For ease in removing the sample vial, the ear 46 may be rotated out of the forward locking slot 16 and pulled through the guide slot. After the sample vial has been removed from the safety syringe, the cover 22 is closed.

When the ear 46 is moved to the rear of the guide slot 14 it encounters a rear locking slot 18. The rear locking slot 18 is angled so that when the ear 46 is in a fully retracted position, the base 30 will tilt to a certain angle. In this way the entire needle assembly, since it is completely inside the body 10, will slant to one side. This easily identifies the safety syringe as having been used and prevents accidental reuse of the safety syringe and prevents the front needle 42 from protruding from the body 10 of the safety syringe. Since the base 30 comprises a base slot 34, the wall of the base 30 is flexible and therefore will bend allowing the base 30 to tilt at an angle.

As a result, the safety of medical personnel is ensured against accidentally being poked by the tip of the front needle. Also, since the cover closes the rear end of the body, medical personal are not in danger of being poked by the tip of the rear needle. Additionally, used syringes are easily identified and disposed of thereby preventing accidental reuse and ensuring the safety of patients.

In an embodiment of the present invention, the rear locking slot 18 is angled to the left or right allowing the needle to tilt to the left or right. In other embodiments of the present invention, the rear locking slot 18 is not angled. Therefore, the base and needle assembly will not tilt to an angle when fully retracted. However, the base and needle assembly will still be locked in a retracted position to prevent the front needle from protruding from the body.

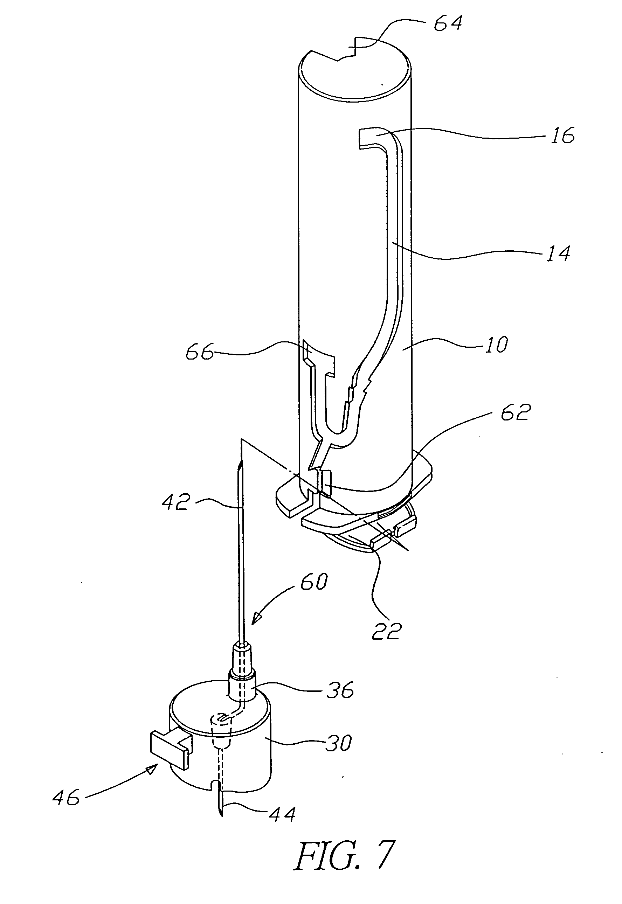

Refer to Figure 7, which is an assembly drawing showing a safety syringe with alternative guide slot according to an embodiment of the present invention.

In Figure 7, an alternative guide slot shape is shown. In this embodiment of the present invention the guide slot 14 begins in a U-shaped path near the rear lock slot 66. After the zig-zag section, the guide slot 14 continues in an arc-shaped path ending in a forward lock slot 16. In this way, the ear 44 can be easily moved from a retracted position to a fully locked extended position is a single smooth movement. Additionally, the forward lock slot 16 may be positioned to the right instead of to the left as shown in Figure 7.

Additionally, the body 10 further comprises a tab and slot 62 is provided to allow the internal components of the safety syringe to be easily assembled inside the body 10. The tab and slot 62 allow the wall of the body 10 to be flexible during assembly. Once the internal components are assembled into the body 10, the tab and slot 62 are pressed together to increase the rigidity of the wall of the body 10. Since the tab and slot 62 is provided, the ear 46 is produced as a part of the base 30 and is not removable. However, the previously described removably attached ear 46 and base 30 may be used with bodies 10 having the tab and slot 62.

Also shown in Figure 7 is an alternative needle assembly 60. In this embodiment the needle assembly 60 comprises a front needle 42 and a bent rear needle 44. To facilitate this type of needle assembly 60, the needle mount 36 is positioned to one side of the base 30. Since the front needle 42 is positioned to one side, the opening 64 is enlarged.

In the previous description, the nature of the guide slot requires the ear to be moved along the guide slot until it reaches the end and then turned to one side in the forward lock slot. Embodiments of the present invention allow the ear to smoothly travel the length of the guide slot to the forward lock slot in one motion, thereby increasing the functionality and ease of use of the safety syringe for medical personnel.

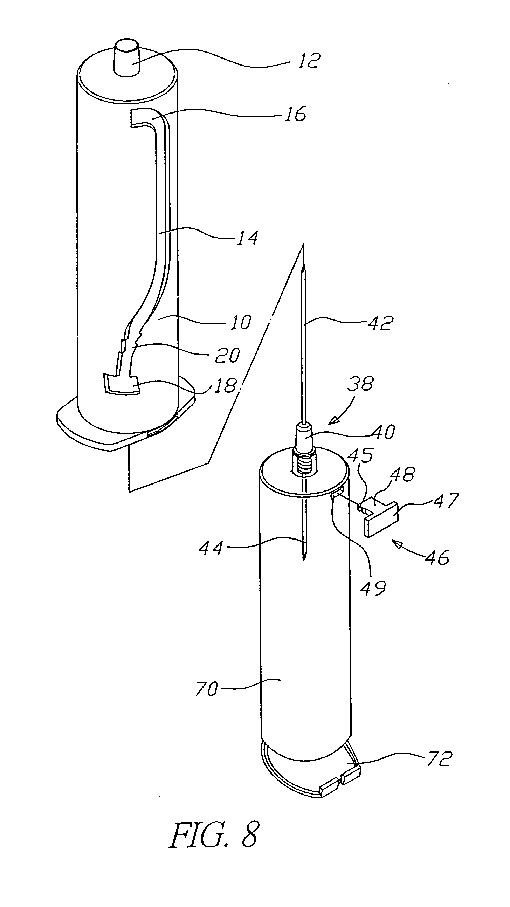

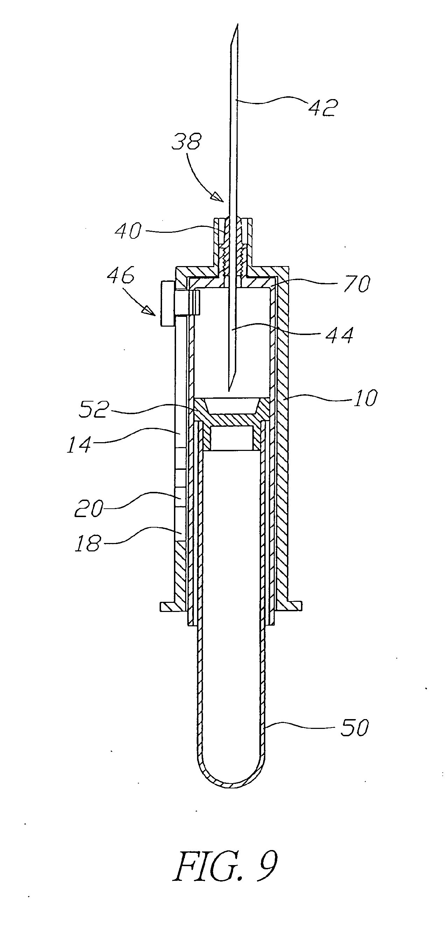

Refer to Figure 8, which is a drawing illustrating a safety syringe with extended base according to an embodiment of the present invention, and Figure 9, which is a plan view drawing showing a safety syringe with extended base according to an embodiment of the present invention.

In an embodiment of the present invention, the base is an extended base 70. The function and use are similar to the previously described embodiments. However, in this embodiment the cover 72 is attached to the base 70 instead of to the body 10. The extended base 70 allows for varying sizes and lengths of sample vials to be used as well as providing coverage around the sample vial when the base 70 is retracted to the rear of the guide slot. After the blood sample has been taken, the needle assembly can be retracted to the fully retracted position in the rear lock slot.

The base 70 is of an appropriate diameter so that the sample vial 50 enters the base 70. The length of the extended base 70 may be made to an appropriate length that is suitable for the sample vial size 50 to be used.

Since this embodiment of the present invention may incorporate the safety features described above such as the cover, rear locking slot, saw tooth section, and forward locking slot, the safety of medical personnel and patients is ensured.

Additionally, as with other embodiments of the present invention, the guide slot, forward lock slot, and rear lock slot may be shaped in various designs as suitable.

In some embodiments of the present invention the slot and tab are not provided. Therefore, the body is a solid wall at the rear of the body instead of being split.

In order to facilitate assembly of the safety syringe the ear 46 is attachable to the base 70 instead of being manufactured as a part of the base. The needles are attached to the base 30 and inserted into the body 10 and the ear 46 is attached to the base 30 from the outside of the body 10. The ear 46 and base 30 comprise appropriate mating tabs or slots to allow the ear 46 to be firmly attached to the base 30. For example, the base may have a slot 49 and the ear 46 a tab 48 or the base may have a tab and the ear a slot. Once assembled, the function and use of the safety syringe is as previously described.

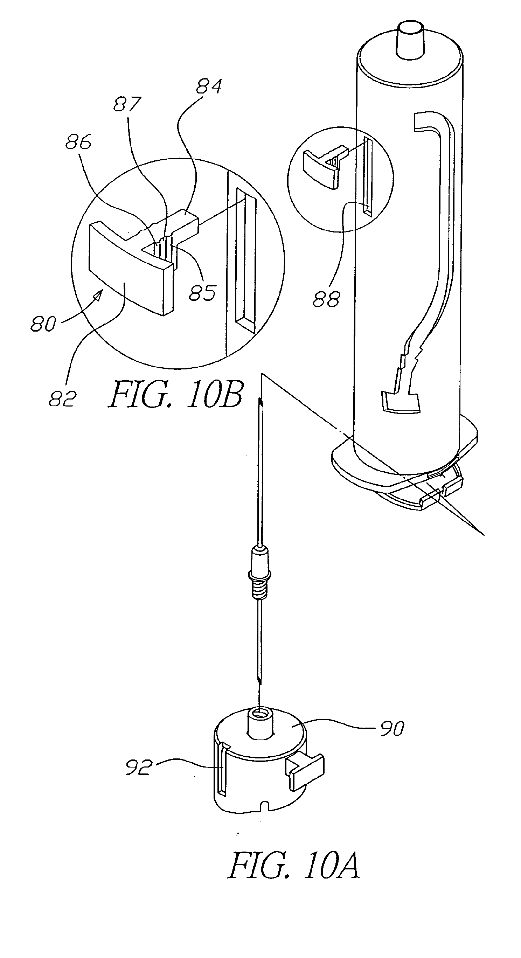

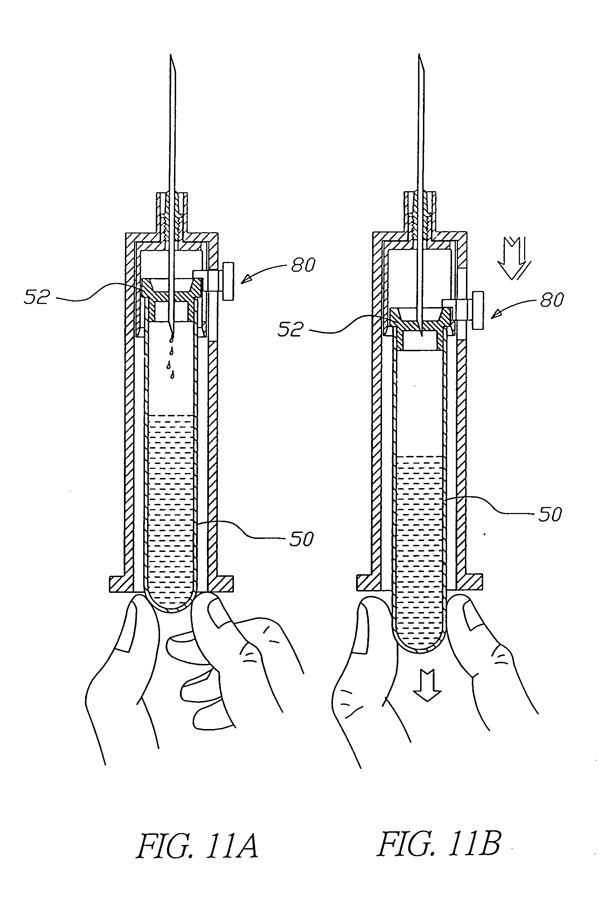

Refer to Figure 10A, which is a drawing illustrating a safety syringe with vial removal knob according to an embodiment of the present invention, Figure 10B, which is an enlarged view of the vial removal knob according to an embodiment of the present invention, Figure 11A, which is a plan view drawing illustrating a safety syringe with vial removal knob according to an embodiment of the present invention, and Figure 11B, which is a plan view drawing illustrating a safety syringe with vial removal knob according to an embodiment of the present invention.

When certain sample vials are used with the safety syringe, removal of the sample vial after the blood sample is taken, may be difficult. Due to the short length of the sample vial, the vial may not extend through the rear opening of the syringe tube or body.

Therefore, in embodiments of the present invention a vial removal knob 80 is provided. The vial removal knob 80 comprises a face 82, a removal arm 84, two raised retainers 85, 86, and a slide 87.

The vial removal knob 80 is attached to a mating slot 88 in the syringe body. By snapping the vial removal knob 80 into the mating slot 88, the nature of the two raised retainers 85 and 86 on either side of the slide 87 hold the vial removal knob 80 securely to the syringe. At the same time this arrangement allows the vial removal knob 80 to slide up and down the mating slot 88 as needed.

In addition a removal opening 92 is provided in the base 90. The removal arm 84 of the vial removal knob 80 extends through the removal opening 92 of the base 90. Once the vial removal knob 80 is snapped into the mating slot 88, the sample vial 50 may be inserted into the syringe body. The removal arm 84 of the vial removal knob 80 rests atop the top of the sample vial 50 during use. After the sample has been taken, the vial removal knob 80 is pushed down thereby causing the removal arm 80 to force the sample vial 50 down, thus dislodging the sample vial 50 and allowing the sample vial 50 to be easily grasped and removed from the safety syringe.

Since the removal opening 92 is provided, the vial removal knob 80 is allowed sufficient movement to dislodge the sample vial 50 without requiring movement of the base 90 or needle assembly.

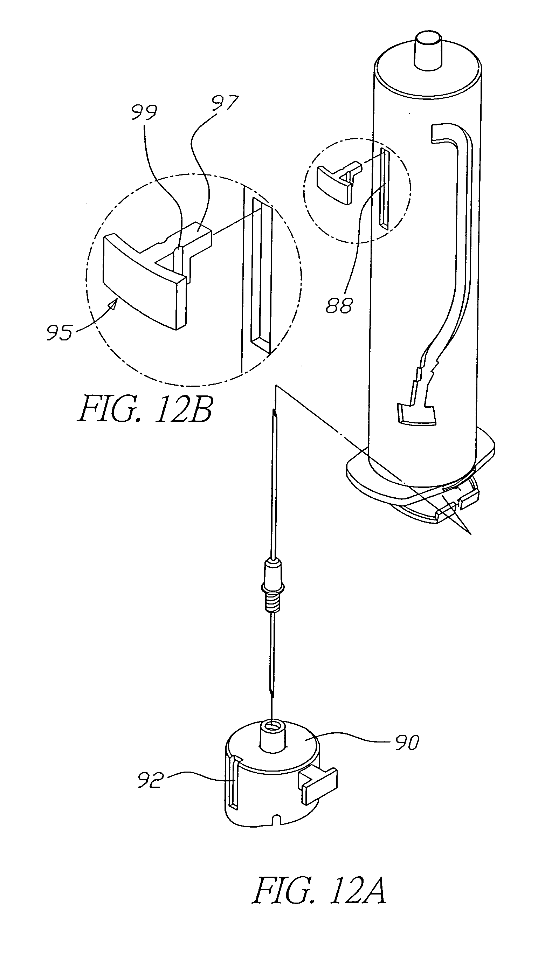

Refer to Figure 12A, which is a drawing illustrating a safety syringe with vial removal knob according to an embodiment of the present invention, and Figure 12B, which is an enlarged view of the vial removal knob according to an embodiment of the present invention.

In embodiments of the present invention, an alternative vial removal knob retaining method is provided. In this embodiment, the vial removal knob 95 is attached to the mating slot 88 by means of a groove 99 in the arm 97 of the vial removal knob 95.

The vial removal knob 95 is snapped into the mating slot 88, and the nature of the groove 99 holds the vial removal knob 95 securely to the syringe. At the same time this arrangement allows the vial removal knob 95 to slide up and down the mating slot 88 as needed.

The function and operation of the vial removal knob 95 is as described above.

Therefore, the present invention provides a safety syringe with retractable needle and locking extended and retracted positions that ensures the safety of medical personnel and patients as well as an improved ease of use.

It will be apparent to those skilled in the art that various modifications and variations can be made to the present invention without departing from the scope or spirit of the invention.

In view of the foregoing, it is intended that the present invention cover modifications and variations of this invention provided they fall within the scope of the invention and its equivalent.

## Claims

1. A safety syringe for extracting blood samples comprising:
a needle mounting base;
a hollow needle comprising a front tip and a rear tip removably attached to the needle mounting base;
a body for holding the needle mounting base comprising;
a front opening;
a rear opening; and
a guide slot;
whereby a portion of the needle mounting base protrudes through the guide slot to allow the needle mounting base to be moved along the guide slot through the body so that the front tip of the hollow needle can be extended through the front opening of the body and can be retracted back into the body.

2. The safety syringe for extracting blood samples of claim 1, whereby the guide slot further comprises a forward lock slot for holding the needle mounting base in an extended position.

3. The safety syringe for extracting blood samples of claim 2, whereby the forward lock slot allows the front tip of the hollow needle to rotate left or right.

4. The safety syringe for extracting blood samples of claim 2, whereby the forward lock slot is shaped to allow the front tip of the hollow needle to rotate left.

5. The safety syringe for extracting blood samples of claim 2, whereby the forward lock slot is shaped to allow the front tip of the hollow needle to rotate right.

6. The safety syringe for extracting blood samples of claim 1, further comprising:
a vacuum vial with seal for containing the blood sample;
whereby the vacuum vial is inserted into the body through the rear opening until the rear tip of the hollow needle punctures the seal so that blood can flow from a patient through the hollow needle and into the vacuum vial.

7. The safety syringe for extracting blood samples of claim 1, the guide slot further comprising a rear lock slot for holding the needle mounting base in a retracted position.

8. The safety syringe for extracting blood samples of claim 7, whereby the rear lock slot is angled to allow the hollow needle to tilt at an angle when in the retracted position.

9. The safety syringe for extracting blood samples of claim 1, the guide slot further comprising a saw tooth section to hinder the needle mounting base from inadvertently moving through the guide slot.

10. The safety syringe for extracting blood samples of claim 1, whereby the body further comprises a cover for closing and opening the rear opening of the body.

11. The safety syringe for extracting blood samples of claim 1, whereby the hollow needle is a two piece needle.

12. The safety syringe for extracting blood samples of claim 1, whereby the needle mounting base further comprises a base slot for increasing the flexibility of the needle mounting base.

13. The safety syringe for extracting blood samples of claim 1, whereby the body further comprises a slot and tab at the rear opening of the body;
whereby when the slot and tab are open, the needle mounting base is easily inserted into the body; and
whereby which the slot and tab are closed, the rigidity of the body is increased.

14. The safety syringe for extracting blood samples of claim 1, whereby the needle mounting base further comprises an attachable ear that extends through the guide slot for moving the needle mounting base through the body.

15. The safety syringe for extracting blood samples of claim 1, whereby the guide slot comprises a U-shaped section and an arc-shaped section.

16. The safety syringe for extracting blood samples of claim 1, whereby the guide slot is arc-shaped.

17. The safety syringe for extracting blood samples of claim 1, whereby the needle mounting base extends through the rear opening of the base.

18. The safety syringe for extracting blood samples of claim 17, whereby the needle mounting base further comprises a cover for closing and opening an end of the needle mounting base.

19. The safety syringe for extracting blood samples of claim 1, further comprising a vial removal knob for facilitating removal of a vacuum vial.

20. The safety syringe for extracting blood samples of claim 19, whereby the vial removal knob comprises:
a face for allowing a user to operate the vial removal knob;
a removal arm connected to the face for applying pressure to a vacuum vial; and
a retainer between the face and the removal arm for holding the vial removal knob to the body of the safety syringe;
whereby when the vial removal knob is moved, the removal arm dislodges the vacuum vial and allows easy removal of the vacuum vial from the safety syringe.

21. The safety syringe for extracting blood samples of claim 20, further comprising:
a mating slot in the body of the safety syringe; and
a removal opening in the base;
whereby the removal arm extends through the mating slot and the removal opening; and
whereby the retainer removably attaches to the mating slot and allows the vial removal knob to move without disengaging from the body.

22. A safety syringe for extracting blood samples comprising:
a needle mounting base further comprising an ear;
a hollow needle comprising a front tip and a rear tip removably attached to the needle mounting base;
a body for holding the needle mounting base comprising;
a front opening;
a rear opening; and
a guide slot;
whereby the ear of the needle mounting base extends through the guide slot to allow the needle mounting base to be moved along the guide slot through the body so that the front tip of the hollow needle can be extended through the front opening of the body and can be retracted back into the body;
a forward lock slot for holding the needle mounting base in an extended position; and
a rear lock slot for holding the needle mounting base in a
retracted position.

23. The safety syringe for extracting blood samples of claim 22, whereby the forward lock slot is shaped to allow the front tip of the hollow needle to rotate left or right.

24. The safety syringe for extracting blood samples of claim 22, further comprising:
a vacuum vial with seal for containing the blood sample;
whereby the vacuum vial is inserted into the body through the rear opening until the rear tip of the hollow needle punctures the seal so that blood can flow from a patient through the hollow needle and into the vacuum vial.

25. The safety syringe for extracting blood samples of claim 22, whereby the rear lock slot is angled to allow the hollow needle to tilt at an angle when in the retracted position.

26. The safety syringe for extracting blood samples of claim 22, the guide slot further comprising a saw tooth section to hinder the needle mounting base from inadvertently moving through the guide slot.

27. The safety syringe for extracting blood samples of claim 22, whereby the body further comprises a cover for closing and opening the rear opening of the body.

28. The safety syringe for extracting blood samples of claim 22, whereby the hollow needle is a two piece needle.

29. The safety syringe for extracting blood samples of claim 22, whereby the needle mounting base further comprises a base slot for increasing the flexibility of the needle mounting base.

30. The safety syringe for extracting blood samples of claim 22, whereby the body further comprises a slot and tab at the rear opening of the body;
whereby when the slot and tab are open, the needle mounting base is easily inserted into the body; and
whereby which the slot and tab are closed, the rigidity of the body is increased.

31. The safety syringe for extracting blood samples of claim 22, whereby the guide slot comprises a U-shaped section and an arc-shaped section.

32. The safety syringe for extracting blood samples of claim 22, whereby the guide slot is arc-shaped.

33. The safety syringe for extracting blood samples of claim 22, whereby the needle mounting base extends through the rear opening of the base.

34. The safety syringe for extracting blood samples of claim 33, whereby the needle mounting base further comprises a cover for closing and opening an end of the needle mounting base.

35. The safety syringe for extracting blood samples of claim 22, whereby the ear is removably attached to the needle mounting base.

36. The safety syringe for extracting blood samples of claim 22, further comprising a vial removal knob for facilitating removal of a vacuum vial.

37. The safety syringe for extracting blood samples of claim 36, whereby the vial removal knob comprises:
a face for allowing a user to operate the vial removal knob;
a removal arm connected to the face for applying pressure to a vacuum vial; and
a retainer between the face and the removal arm for holding the vial removal knob to the body of the safety syringe;
whereby when the vial removal knob is moved, the removal arm dislodges the vacuum vial and allows easy removal of the vacuum vial from the safety syringe.

38. The safety syringe for extracting blood samples of claim 37, further comprising:
a mating slot in the body of the safety syringe; and
a removal opening in the base;
whereby the removal arm extends through the mating slot and the removal opening; and
whereby the retainer removably attaches to the mating slot and allows the vial removal knob to move without disengaging from the body.
